(19) Europäisches Patentamt
European Patent Office
Office européen des brevets

(11) Veröffentlichungsnummer: **0 180 707**
**A1**

(12) **EUROPÄISCHE PATENTANMELDUNG**

(21) Anmeldenummer: 85105968.3

(22) Anmeldetag: 15.05.85

(51) Int. Cl.⁴: C 07 D 231/52

(30) Priorität: 04.10.84 DE 3436383

(43) Veröffentlichungstag der Anmeldung: 14.05.86 Patentblatt 86/20

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Naab, Paul, Dr.
Schenkstrasse 32b
D-5600 Wuppertal 21(DE)

(72) Erfinder: Ertel, Werner, Dr.
Kyffhäuser Strasse 37
D-5600 Wuppertal 1(DE)

(54) **Verfahren zur Herstellung von Pyrazolonderivaten.**

(57) Die vorliegende Erfindung betrifft ein verbessertes Syntheseverfahren für 1-substituierte 3-Amino-pyrazolone-(5), wobei in einem ersten Schritt ein Hydrazinderivat mit β-Amino-β-alkoxyacrylsäurealkylester sauer kondensiert und das Zwischenprodukt in einem zweiten Schritt in stark basischem Milieu bei Temperaturen unter 10°C einer Ringschlußreaktion unterworfen wird.

EP 0 180 707 A1

Croydon Printing Company Ltd.

BAYER AKTIENGESELLSCHAFT 5090 Leverkusen, Bayerwerk

Konzernverwaltung RP Sft/lic
Patentabteilung

## Verfahren zur Herstellung von Pyrazolonderivaten

Die vorliegende Erfindung betrifft ein verbessertes Syntheseverfahren für 1-substituierte 3-Amino-pyrazolone-(5), wobei in einem ersten Schritt ein Hydrazinderivat mit ß-Amino-ß-alkoxyacrylsäurealkylester sauer kondensiert und das Zwischenprodukt in einem zweiten Schritt in stark basischem Milieu bei Temperaturen unter 10°C einer Ringschlußreaktion unterworfen wird.

3-Amino-pyrazolon-(5)-Derivate und Verfahren zu ihrer Herstellung werden in DE-OS 2 319 280 beschrieben. Die Verbindungen weisen einen ausgeprägten diuretischen und antihypertensiven Effekt auf, wobei sich das 3-Amino-1-($\alpha$-methyl-4-chlorbenzyl)-pyrazolon-(5) ("Muzolimin") als besonders wertvoll erwiesen hat. Hinsichtlich der pharmakologischen Wirkung von Muzolimin sei z. B. auf Recent Advances in Diuretic Theraphy: Proc. of the Sec. Int. Edrul Symposium (1983), herausgegeben von V.E.Andreucci, Excerpta Medica, Amsterdam, auf Clin. Nephrol. 19 (1983): Suppl. 1, Loew et al in Eur. J.Clin. Pharmacol., 12 (1977), S. 341-344 und die dort zitierte Literatur hingewiesen.

Die Aminopyrazolone werden gemäß DE-OS 2 319 280 durch Umsetzung des entsprechenden Hydrazins mit einem geeigneten Essigsäurederivat, gegebenenfalls in Gegenwart inerter Lösungsmittel und basischer oder saurer Katalysatoren wie Alkali- und Erdalkalihydroxide und -carbonate oder wie Halogenwasserstoffsäuren, Schwefelsäure oder Sulfonsäuren, bei Temperaturen zwischen 10 und 200°C hergestellt. Muzolimin wird gemäß Beispiel 9 von DE-OS 2 319 280 erhalten, indem man ß-Amino-ßethoxyacrylsäureethylester und p-Toluolsulfonsäure in absolutem Alkohol mit α-Methyl-3,4-dichlorbenzylhydrazin tropfenweise versetzt, die Mischung rührt, über Nacht stehenläßt und den Alkohol abdampft. Der Rückstand wird in 2n-NaOH gelöst und von Verunreinigungen durch Extrahieren mit Ether befreit. Die wäßrige Phase wird mit Essigsäure auf pH 5 gebracht, das anfallende Öl in Methylenchlorid aufgenommen, getrocknet und das Lösungsmittel abgedampft.

Das in DE-OS 2 319 280 beschriebene Verfahren hat eine Reihe von Nachteilen: Das erhaltene Produkt ist gelblich verfärbt und die Ausbeuten sind - vor allem bei Übertragung in den technischen Maßstab - gering (ca. 20-25 %). Es war somit Aufgabe der vorliegenden Erfindung, ein technisch einfaches Syntheseverfahren für Aminopyrazolone zu finden, das hohe Ausbeuten von farblich einwandfreiem Produkt auch in großtechnischen Ansätzen ermöglicht. Diese Aufgabe wird mit dem erfindungsgemäßen Verfahren gelöst.

Gegenstand der vorliegenden Erfindung ist ein Verfahren zur Herstellung von Pyrazolonderivaten der Formel

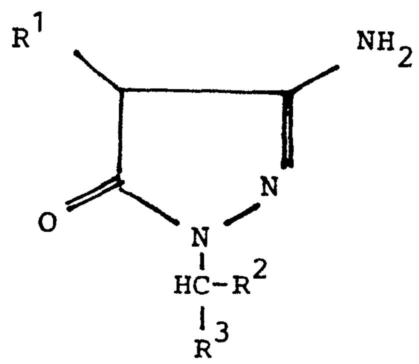

(I)

welcher

$R^1$ für Wasserstoff, Alkyl, Alkenyl oder gegebenenfalls substituiertes Aryl oder Aralkyl steht,

$R^2$ einen Alkylrest darstellt und

$R^3$ für einen gegebenenfalls substituierten Arylrest, der bis zu zwei gleiche oder verschiedene Substituenten aus der Gruppe Halogen, Trifluormethyl, Alkyl, Alkenyl, Alkoxy enthält oder einen Alkylamino-, Trifluormethoxy-, Nitro-, Cyano-, Carbonamino-, Sulfonamido- oder $SO_n$-Alkylrest (n = 0 bis 2), gegebenenfalls zusammen mit 1 oder 2 Substituenten aus der Gruppe Alkyl, Alkenyl, Alkoxy, Halogen oder Trifluormethyl enthält,
wobei gegebenenfalls zwei Substituenten am Arylrest gemeinsam einen verzweigten oder unverzweigten, gesättigten oder ungesättigten, 5- bis 7-gliedrigen isocyclischen oder heterocyclischen Ring bilden, der seinerseits 1 bis 2 Sauerstoff oder Schwefelatome enthalten kann, steht,

indem man ein Hydrazin der Formel

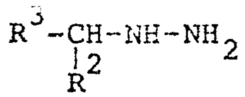

(II)

in welcher $R^2$ und $R^3$ die oben angegebene Bedeutung haben, mit einem Essigsäurederivat der Formel

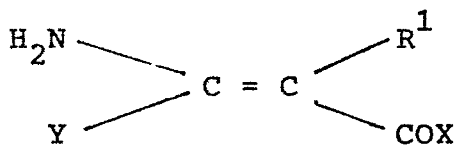

(III)

in welcher

$R^1$ die oben angegebene Bedeutung hat,
X für einen Hydroxy-, Alkoxy-, Aralkoxy-, Amino- oder Alkylaminorest und
Y für einen Alkoxy-, Aryloxy-, Aralkoxy-, Alkylmercapto- oder Aralkylmercaptorest oder eine Aminogruppe steht,

in Gegenwart basischer oder saurer Katalysatoren sowie gegebenenfalls inerter Lösungsmittel umsetzt, dadurch gekennzeichnet, daß man

a) das Essigsäurederivat (III) in Form seines Salzes mit einer starken Säure einsetzt;
b) das Salz des Essigsäurederivats (III) mit dem Hydrazin (II) in einem ersten Schritt in Gegenwart von 0.1-2.0 Äquivalenten einer schwachen Base in einem vorzugsweise wäßrigen Reaktionsmedium kondensiert;

0180707

c) das erhaltene Zwischenprodukt mit einer konzentrierten wäßrigen Lösung einer starken Base weiter kondensiert, wobei die Reaktionstemperatur unter 10°C gehalten wird; und

d) das Reaktionsprodukt durch Einstellen eines pH-Werts von 0 bis 3,5 mittels einer starken wäßrigen Säure ausfällt.

Bevorzugt wird das Zwischenprodukt der ersten Stufe des erfindungsgemäßen Verfahrens noch im sauren Milieu durch Extraktion mittels eines organischen Lösungsmittels von Verunreinigungen und Nebenprodukten befreit. Vorzugsweise wird das Zwischenprodukt aus dem Reaktionsgemisch nicht isoliert, sondern direkt in Form einer Eintopfreaktion in der zweiten Stufe weiter umgesetzt.

In der obigen Formel (I) stehen vorzugsweise

$R^1$ für Wasserstoff, einen Alkyl- oder Alkenylrest mit bis zu 4 C-Atomen, eine Phenyl- oden Benzylgruppe, welche gegebenenfalls durch eine Alkoxygruppe mit 1 bis 2 C-Atomen substituiert sind, besonders bevorzugt für Wasserstoff,

$R^2$ für einen Alkylrest mit 1 bis 4 Kohlenstoffatomen, besonders bevorzugt Methyl, und

$R^3$ für einen Phenyl- oder Naphthylrest, insbesondere für einen Phenylrest, der durch 1 bis 2 geradkettige oder verzweigte Alkyl- oder Alkenylreste mit bis zu 8 Kohlenstoffatomen, insbesondere durch 1 bis 2 Alkylreste mit 1 bis 4 Kohlenstoffatomen, oder vorzugsweise durch 1-2 Alkoxyreste mit bis zu 6 Kohlenstoffatomen, insbesondere mit bis zu 4 Kohlenstoffatomen, oder durch

1 Cycloalkyl- oder Cycloalkenylrest mit 5 bis 7 Kohlenstoffatomen, oder durch 1 bis 2 Halogenatome wie Fluor, Chlor oder Brom oder durch 1 bis 2 Trifluormethylreste oder durch eine Trifluormethoxy-, Nitro-, Cyanogruppe oder eine Dialkylamino-, Carbonamido- oder Sulfonamidogruppe, deren Stickstoffatom durch 1 oder 2 geradkettige oder verzweigte Alkylgruppen mit jeweils 1 bis 4 C-Atomen substituiert sein kann und wobei die vorerwähnten Alkylgruppen gemeinsam mit dem Stickstoffatom einen 5- bis 7-gliedrigen heterocyclischen Ring bilden können, der ein Sauerstoffatom als zusätzliches Heteroatom enthalten kann, oder durch eine $SO_n$-Alkyl-Gruppe, wobei n für 0 bis 2, insbesondere für 0 oder 2 steht, und der Alkylrest geradkettig oder verzweigt ist und 1 bis 4 Kohlenstoffatome enthält, substituiert ist und wobei 2 Substituenten am Phenyl- oder Naphthylring gemeinsam einen verzweigten oder unverzweigten, gesättigten oder ungesättigten 5- bis 7-gliedrigen, isocyclischen oder heterocyclischen Ring, der 1 Schwefel- oder 1 bis 2 Sauerstoffatome enthalten kann, bilden können.

In Formel (III) stehen vorzugsweise

X für Hydroxyl, eine gegebenenfalls verzweigte Alkoxygruppe mit 1 bis 6 C-Atomen, insbesondere 1 bis 3 C-Atomen, eine Benzyloxy-, eine Amino-, Alkylamino- oder Dialkylaminogruppe mit jeweils 1 bis 4 C-Atomen pro Alkylgruppe und

Y für einen Alkoxy- oder Alkylmercaptorest mit jeweils 1 bis 6 C-Atomen in der Alkylgruppe, insbesondere einen Alkoxyrest mit 1 bis 3 C-Atomen, oder einen Benzyloxy-, Phenoxy-, Benzylmercaptorest oder eine Aminogruppe.

Beispiele für erfindungsgemäß zu synthetisierende Verbindungen der Formel (I) bzw. Ausgangsverbindungen der Formeln (II) und (III) sind in DE-OS 2 319 280 angegeben.

Verbindung (III), im folgenden "Imidoester" genannt, wird im erfindungsgemäßen Verfahren als Salz einer starken Säure ($K_A$ >1), vorzugsweise einer Mineralsäure (insbesondere Salzsäure), eingesetzt.

Das Salz des Imidoesters wird in der ersten Stufe des erfindungsgemäßen Verfahrens mit dem Hydrazin (II) vorzugsweise in einem wäßrigen Reaktionsmedium in Gegenwart von 0.1-2.0 Äquivalent einer schwachen Base umgesetzt, vorzugsweise mit 0.4-0.6 Äquivalent. Geeignete schwache Basen sind z. B. Salze von Carbonsäuren oder Amine.

Bevorzugt sind Salze von Carbonsäuren, insbesondere Na-Acetat. Die Reaktionstemperatur liegt zwischen -20°C und +50°C, vorzugsweise zwischen 10°C und 30°C.

Das Reaktionsgemisch in der ersten Stufe des erfindungsgemäßen Verfahrens kann gegebenenfalls ein mit Wasser mischbares organisches Lösungsmittel enthalten.

0180707

Beispiele hierfür sind Methanol, Ethanol, Isopropanol, Tetrahydrofuran oder Dioxan.

Bevorzugt sind die dem (oder den) Alkoxyrest(en) des Imidoesters entsprechenden Alkohole.

Das Reaktionsgemisch der ersten Stufe des erfindungsgemäßen Verfahrens wird vorzugsweise bei dem oben angegebenen schwach sauren pH durch Extraktion mittels eines organischen Lösungsmittels von Nebenprodukten und nicht umgesetzten Ausgangsprodukten befreit. Geeignete Lösungsmittel sind z. B. Petrolether, Ether, Methylenchlorid oder Toluol.

Bevorzugt ist Toluol.

Nach Abtrennen der organischen Phase des Extraktionsschritts wird die wäßrige Phase vorzugsweise direkt, d. h. ohne Isolierung des Zwischenprodukts, der alkalischen Kondensationsreaktion unterworfen. Dies geschieht durch Zusatz einer konzentrierten starken Base ($K_B > 1$), so daß das Reaktionsgemisch 1 bis 10 Gew.-%, bevorzugt 2 bis 5 Gew.-%, der starken Base enthält. Als starke Basen kommen vorzugsweise Alkali- und Erdalkalihydroxide in Betracht, insbesondere NaOH und KOH. Während der alkalischen Kondensationsreaktion soll die Temperatur des Reaktionsgemisches unter 10°C liegen, vorzugsweise zwischen -15°C und 5°C, insbesondere bei etwa 0°C. Die Reaktionsdauer beträgt vorzugsweise weniger als 1 h, besonders bevorzugt 10 bis 30 Minuten.

Das Reaktionsgemisch wird danach durch Zusatz einer starken Säure (z. B. den oben beim Imidoester-Salz genannten Säuren) auf einen pH-Wert von 0 bis 3,5, vorzugsweise 1 bis 2,5, insbesondere etwa 1.5 - 2, eingestellt, wobei das Reaktionsprodukt ausfällt. Das Rohprodukt kann durch Umkristallisieren, z. B. aus wäßrigem Alkohol, weiter gereinigt werden.

Das erfindungsgemäße Syntheseverfahren weist gegenüber den in DE-OS 2 319 280 beschriebenen Verfahren eine Reihe von Vorteilen auf:

1) Der Imidoester wird direkt als Salz einer starken Säure, d. h. in der großtechnisch anfallenden Form eingesetzt; der Imidoester braucht somit nicht rein dargestellt zu werden, was 1 Stufe einspart.

2) Nach der sauren Kondensationsstufe wird kein organisches Lösungsmittel abdestilliert, d. h. es wird Zeit und Energie gespart und das Produkt nicht thermisch belastet.

3) Durch das Extrahieren bei saurem pH nach der sauren Kondensationsstufe werden Nebenprodukte und nicht umgesetztes Ausgangsmaterial entfernt.

4) Die alkalische Kondensation (Cyclisierung) erweist sich bei Temperaturen unter 10°C besser beherrschbar und reproduzierbar.

5) Durch das Einstellen eines stark sauren pH nach der alkalischen Kondensation werden Nebenprodukte wie z. B. 3-N(3.4-dichlor-α-methyl-benzyl)-acet-amidrazon IV

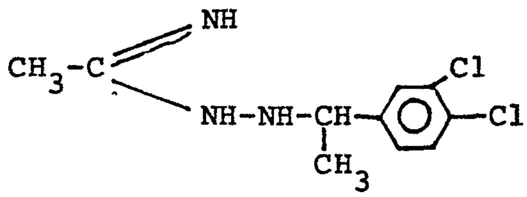

IV

wesentlich besser entfernt; dies ist beim Ansäuern mittels Essigsäure nicht der Fall.

6) Das Reaktionsprodukt besitzt wesentlich bessere Farbqualität und wird in Ausbeuten von über 50 % erhalten.

7) Das gesamte Syntheseverfahren ist zeitlich schneller und technisch einfacher durchführbar.

Beispiel 1 (erfindungsgemäß)

a) Herstellung des Hydrazins (II):

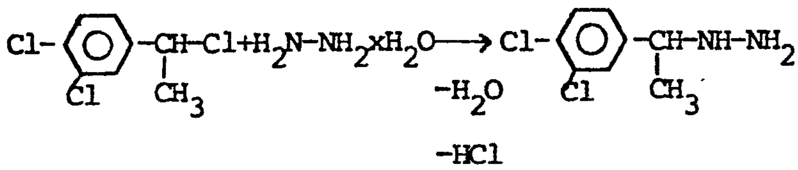


1,2-Dichlor-4-(1-chlorethyl)-benzol wird mit Hydrazinhydrat in einem mit Wasser mischbaren Lösungsmittel, z. B. Ethanol, durch Erhitzen zu N-/‾1-(3,4-Dichlorphenyl)-ethyl_7-hydrazin umgesetzt. Nach Abtrennen des gebildeten Hydrazinhydrochlorids wird das Reaktionsgemisch mit Wasser gewaschen. Das so erhaltene Hydrazin kann direkt, d. h. ohne weitere Reinigung, weiterverarbeitet werden.

b) Herstellung von Muzolimin:

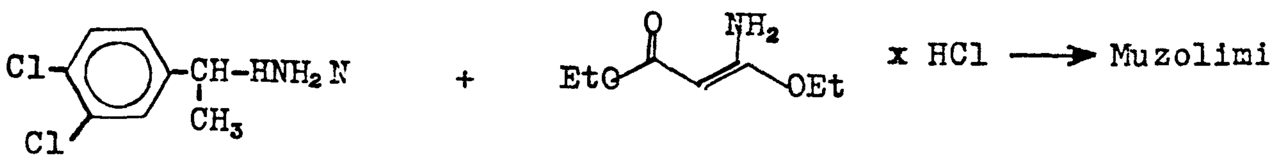


142 Gewichtsteile 2-Methyl-3,4-dichlorbenzylhydrazin (Rohmaterial aus Stufe a) werden mit 60 Volumenteilen Wasser versetzt. Anschließend gibt man 69,5 Gewichtsteile Natriumacetat-Trihydrat dazu und innerhalb von 10 Minuten 103,5 Gewichtsteile Imidoesterhydrochlorid. Nun wird 25 Minuten nachgerührt. Dann gibt man 385 Volumenteile Ethanol dazu, rührt 10 Minuten durch und

filtriert vom Ungelösten ab. Das Filtrat wird mit 375 Volumenteilen Wasser und 600 Volumenteilen Toluol verrührt und die organische Phase abgetrennt. Die wäßrige Phase wird nochmals mit 600 Volumenteilen Toluol verrührt und nach Abtrennen des Toluols auf -5°C abgekühlt. Nun wird innerhalb von 10 Minuten unter Kühlung eine Mischung aus 82,8 Gewichtsteilen 45 %iger Natronlauge und 128,1 Volumenteilen Wasser dazugegeben und weitere 10 Minuten bei 5°C gerührt. Diese Lösung wird dann mit 140 Volumenteilen eines Gemisches aus 154,7 Gewichtsteilen Salzsäure (rein, 37 %ig) und 46,5 Volumenteilen Wasser auf pH 1,5 gestellt, wobei das 3-Amino-1-(3,4-dichlor - $\alpha$-methylbenzyl)-2-pyrazolin-5-on ausfällt. Es werden noch 750 Volumenteile Wasser dazugegeben und das Kristallisat isoliert. Das feuchte Rohmaterial wird aus Branntwein/Wasser umkristallisiert und getrocknet. Man erhält 77 Gewichtsteile 3-Amino-1-(3,4-dichlor-$\alpha$-methylbenzyl)-2-pyrazolin-5-on vom Schmelzpunkt 137°C (51,8 % der Theorie).

<u>Beispiel 2</u> (Vergleich)

a) Herstellung von Imidoester

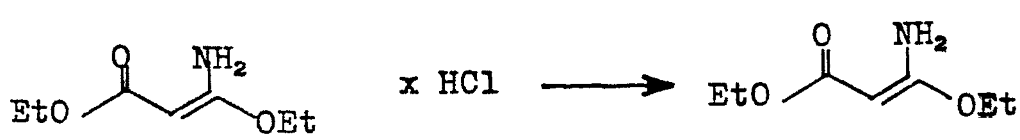


109.2 g Natriumhydrogencarbonat werden in 1,16 l Wasser gelöst und mit 280 ml Toluol überschichtet. Nun werden 195,7 g Imidoesterhydrochlorid portionsweise

dazugegeben und 5 Minuten nachgerührt, bis keine Kohlendioxidentwicklung mehr zu beobachten ist. Die Wasserphase wird abgetrennt und erneut mit 140 ml Toluol extrahiert. Die vereinigten Toluolphasen werden mit Natriumsulfat getrocknet, das Natriumsulfat abgesaugt und das Toluol am Rotationsverdampfer eingedampft. Man erhält 154,2 g (96,9 % der Theorie) an rohem Imidoester. Der Imidoester wurd im Hochvakuum destilliert: 129,6 g (Kp. = 59-63°C; 81,4 % der Theorie).

b) Herstellung von Muzolimin gemäß DE-OS 2 319 280:

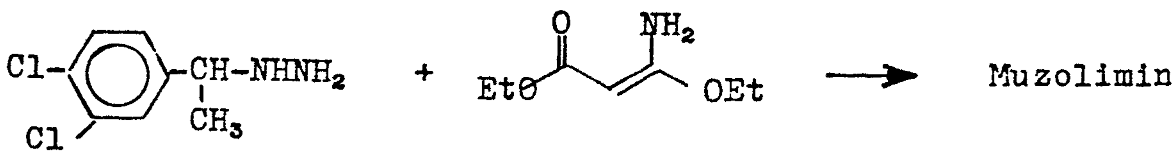

Zu einer Lösung von 31,8 g Imidoester und 1,5 g para-Toluolsulfonsäure in 150 ml Ethanol wurde innerhalb von 18 Minuten 41 g α-Methyl-3,4-dichlorbenzylhydrazin (roh), gelöst in 50 ml Ethanol zugetropft (Temperatur stieg auf 27°C; die Lösung wurde trübe). Anschließend rührte man noch 2 Stunden und ließ über Nacht stehen (Lösung trübe, Feststoff). Am nächsten Morgen wurde das Lösungsmittel im Vakuum abgedampft (Bad 25°C; Enddruck 16 mbar; Zeit: 30 Minuten; Rückstand: orangefarbenes Öl, 73,5 g, enthält Feststoff) und der Rückstand in 200 ml 2 n Natronlauge gelöst (Temp. stieg auf 30°C; wieder auf 20°C gekühlt; 10 Minuten gerührt). Die wäßrige Phase wurde 3 mal mit je 100 ml Diethylether extrahiert und nach Abtrennen der wäßrigen Phase mit 30 ml Essigsäure auf pH 5.0 gestellt. Nun gab man 100 ml Methylenchlorid dazu und saugte vom nicht in Lösung gehenden Feststoff ab.

Der Feststoff (24,5 g, entsprechend 45 % der Theorie) wurde 2 mal mit 25 ml Methylenchlorid gespült und die vereinigten, abgetrennten Methylenchloridphasen wurden mit Natriumsulfat getrocknet. Nach Abtrennen des Natriumsulfats wurde das Methylenchlorid im Vakuum abgezogen (Rückstand: zähes Öl; teilweise kristallin, 23,7 g) und der Rückstand mit dem vorher abgesaugten Feststoff aus 35 ml heißem Methanol umkristallisiert. Nach Abkühlen, Absaugen, Waschen mit Methanol und Trocknen konnten 22,8 g (40,5 % der Theorie) kristallines Material isoliert werden.

Das Produkt wies eine gelbliche Verfärbung auf, die auch durch Umkristallisieren aus Ethanol/Wasser nicht beseitigt werden konnte.

Patentansprüche

1) Verfahren zur Herstellung von Pyrazolonderivaten der Formel

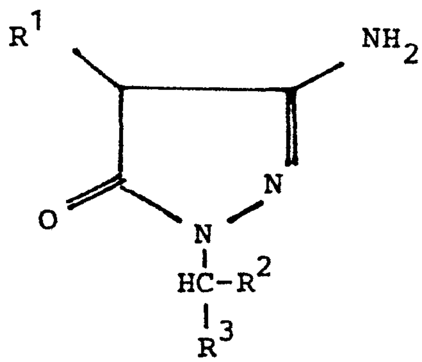

(I)

in welcher

$R^1$ für Wasserstoff, Alkyl, Alkenyl oder gegebenenfalls substituiertes Aryl oder Aralkyl steht,

$R^2$ einen Alkylrest darstellt und

$R^3$ für einen gegebenenfalls substituierten Arylrest, der bis zu zwei gleiche oder verschiedene Substituenten aus der Gruppe Halogen, Trifluormethyl, Alkyl, Alkenyl, Alkoxy enthält oder einen Alkylamino-, Trifluormethoxy-, Nitro-, Cyano-, Carbonamino-, Sulfonamido- oder $SO_n$-Alkylrest (n = 0 bis 2), gegebenenfalls zusammen mit 1 oder 2 Substituenten aus der Gruppe Alkyl, Alkenyl, Alkoxy, Halogen oder Trifluormethyl enthält, wobei gegebenenfalls zwei Substituenten am Arylrest gemeinsam einen verzweigten oder unverzweigten, gesättigten oder ungesättigten, 5- bis 7-gliedrigen isocyclischen oder heterocyclischen Ring bilden, der seinerseits 1 bis 2 Sauerstoff oder Schwefelatome enthalten kann, steht,

indem man ein Hydrazin der Formel

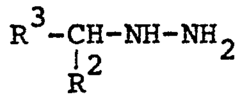

(II)

in welcher $R^2$ und $R^3$ die oben angegebene Bedeutung haben, mit einem Essigsäurederivat der Formel

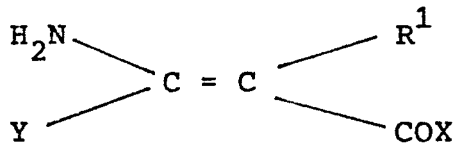

(III)

in welcher

$R^1$ die oben angegebene Bedeutung hat,

X für einen Hydroxy-, Alkoxy-, Aralkoxy-, Amino- oder Alkylaminorest und

Y für einen Alkoxy-, Aryloxy-, Aralkoxy-, Alkylmercapto- oder Aralkylmercaptorest oder eine Aminogruppe steht,

in Gegenwart basischer oder saurer Katalysatoren sowie gegebenenfalls inerter Lösungsmittel umsetzt, dadurch gekennzeichnet, daß man

a) das Essigsäurederivat (III) in Form seines Salzes mit einer starken Säure einsetzt;
b) das Essigsäurederivat (III) mit dem Hydrazin (II) in einem ersten Schritt in Gegenwart einer schwachen Base in einem vorzugsweise wäßrigen Reaktionsmedium kondensiert;

c) das erhaltene Zwischenprodukt mit einer konzentrierten wäßrigen Lösung einer starken Base weiter kondensiert, wobei die Reaktionstemperatur unter 10°C gehalten wird; und

d) das Reaktionsprodukt durch Einstellen eines pH-Werts von 0 bis 3,5 mittels einer starken wäßrigen Säure ausfällt.

2) Verfahren nach Anspruch 1, dadurch gekennzeichnet, daß

$R^1$ für Wasserstoff, einen Alkyl- oder Alkenylrest mit bis zu 4 C-Atomen, eine Phenyl- oder Benzylgruppe, welche gegebenenfalls durch eine Alkoxygruppe mit 1 bis 2 C-Atomen substituiert sind, insbesondere für Wasserstoff, steht.

3) Verfahren nach Anspruch 1 oder 2, dadurch gekennzeichnet, daß

$R^2$ für einen Alkylrest mit 1 bis 4 C-Atomen, bevorzugt für einen Methylrest, steht.

4) Verfahren nach Anspruch 1 bis 3, dadurch gekennzeichnet, daß

$R^3$ für einen Phenyl- oder Naphthylrest, insbesondere für einen Phenylrest steht, der durch 1 bis 2 geradkettige oder verzweigte Alkyl- oder Alkenylreste mit bis zu 8 Kohlenstoffatomen, insbesondere durch 1 bis 2 Alkylreste mit 1 bis 4 Kohlenstoffatomen, oder vorzugsweise durch 1 - 2 Alkoxyreste mit bis zu 6 Kohlenstoffatomen, insbesondere mit bis zu 4 Kohlenstoffatomen, oder durch 1 Cycloalkyl- oder Cycloalkenylrest mit 5 bis 7 Kohlenstoffatomen, oder durch 1 bis 2 Halogenatome wie Fluor, Chlor oder Brom oder durch 1 bis 2 Trifluormethylreste oder durch eine Trifluormethoxy-, Nitro-, Cyanogruppe oder eine Dialkylamino-, Carbonamido- oder Sulfonamidogruppe, deren Stickstoffatom durch 1 oder 2 geradkettige oder verzweigte Alkylgruppen mit jeweils 1 bis 4 C-Atomen substituiert sein kann und wobei die vorerwähnten Alkylgruppen gemeinsam mit dem Stickstoffatom einen 5- bis 7-gliedrigen heterocyclischen Ring bilden können, der ein Sauerstoffatom als zusätzliches Heteroatom enthalten kann,

oder durch eine $SO_n$-Alkyl-Gruppe, wobei n für 0 bis 2, insbesondere für 0 oder 2 steht, und der Alkylrest geradkettig oder verzweigt ist und 1 bis 4 Kohlenstoffatome enthält, substituiert ist und

wobei 2 Substituenten am Phenyl- oder Naphthylring gemeinsam einen verzweigten oder unverzweigten, gesättigten oder ungesättigten 5- bis 7-gliedrigen, isocyclischen oder heterocyclischen Ring, der 1 Schwefel- oder 1 bis 2 Sauerstoffatome enthalten kann, bilden können.

5) Verfahren nach Anspruch 1 bis 4, dadurch gekennzeichnet, daß

X für Hydroxyl, eine gegebenenfalls verzweigte Alkoxygruppe mit 1 bis 6 C-Atomen, eine Benzyloxy-, eine Amino-, Alkylamino- oder Dialkylaminogruppe mit jeweils 1 bis 4 C-Atomen pro Alkylgruppe, insbesondere eine Alkoxygruppe mit 1 bis 3 C-Atomen, und

Y für einen Alkoxy- oder Alkylmercaptorest mit jeweils 1 bis 6 C-Atomen in der Alkylgruppe, einen Benzyloxy-, Phenoxy-, Benzylmercaptorest oder eine Aminogruppe, insbesondere einen Alkoxyrest mit 1 bis 3 C-Atomen, stehen.

6) Verfahren nach Anspruch 1 bis 5, dadurch gekennzeichnet, daß das Produkt der ersten Verfahrensstufe noch im sauren Milieu durch Extraktion mittels eines organischen Lösungsmittels von Verunreinigungen befreit und vorzugsweise ohne Isolierung direkt in Form einer Eintopfreaktion in der zweiten Verfahrensstufe weiter umgesetzt wird.

7) Verfahren nach Anspruch 1 bis 6, dadurch gekennzeichnet, daß Verbindung (III) als Salz einer Mineralsäure, vorzugsweise Salzsäure, eingesetzt wird-

8) Verfahren nach Anspruch 1 bis 7, dadurch gekennzeichnet, daß die Temperatur während der ersten Verfahrensstufe zwischen -20°C und +50°C, vorzugsweise zwischen 10 und 30°C, gehalten wird.

9) Verfahren nach Anspruch 1 bis 8, dadurch gekennzeichnet, daß das Reaktionsgemisch in der zweiten Verfahrensstufe 2 bis 5 Gew.-% eines Alkali- oder Erdalkalihydroxids, vorzugsweise NaOH oder KOH, enthält.

10) Verfahren nach Anspruch 1 bis 9, dadurch gekennzeichnet, daß das Reaktionsgemisch während der zweiten Verfahrensstufe bei -15°C bis +5°C, vorzugsweise etwa 0°C, gehalten wird und die Reaktionszeit weniger als 1 Stunde beträgt.

Europäisches Patentamt

# EUROPÄISCHER RECHERCHENBERICHT

0180707

Nummer der Anmeldung

EP 85 10 5968

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | DE-A-2 230 792 (BAYER)<br>--- | | C 07 D 231/52 |
| A | DE-A-2 319 278 (BAYER)<br>--- | | |
| A | DE-A-2 363 138 (BAYER)<br>--- | | |
| A | DE-A-2 363 139 (BAYER)<br>--- | | |
| D,A | DE-A-2 319 280 (BAYER)<br>----- | | |
| | | | RECHERCHIERTE SACHGEBIETE (Int Cl 4) |
| | | | C 07 D 231/00 |

Der vorliegende Recherchenbericht wurde für alle Patentansprüche erstellt.

| Recherchenort | Abschlußdatum der Recherche | Prüfer |
|---|---|---|
| DEN HAAG | 29-01-1986 | DE BUYSER I.A.F. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82